# EUROPEAN PATENT SPECIFICATION

(11) **EP 1 878 399 B1**
(45) Date of publication and mention of the grant of the patent: **17.04.2013**
(21) Application number: 07013779.9
(22) Date of filing: 13.07.2007
(51) Int. Cl.: A61B 18/04

(54) **Vessel sealing instrument with pre-heated electrodes**
Gefäßversiegelungsinstrument mit vorgeheizten Elektroden
Instrument d'obturation de vaisseaux doté d'électrodes préchauffées

(30) Priority: 14.07.2006 US 487297
(43) Date of publication of application: 16.01.2008
(73) Proprietor: Covidien AG, 8212 Neuhausen am Rheinfall (CH)
(72) Inventor: Odom, Darren, Longmont CO 80501 (US); Weinberg, Craig, Denver CO 80211 (US); Denham, Amy, Denver CO 80209 (US)
(74) Representative: HOFFMANN EITLE

(56) References cited:
- EP-A- 1 632 192
- WO-A-02/067798
- US-A1- 2003 060 816
- US-A1- 2004 078 035
- US-A1- 2004 215 185

## Description

### TECHNICAL FIELD

The present disclosure relates to a forceps used for both endoscopic and open surgical procedures which includes an electrode assembly that allows a user to selectively seal and/or cut tissue.

### BACKGROUND

Open or endoscopic electrosurgical forceps utilize both mechanical clamping action and electrical energy to effect hemostasis. The electrode of each opposing jaw member is charged to a different electric potential such that when the jaw members grasp tissue, electrical energy can be selectively transferred through the tissue. A surgeon can either cauterize, coagulate/desiccate and/or simply reduce or slow bleeding, by controlling the intensity, frequency and duration of the electrosurgical energy applied between the electrodes and through the tissue.

Certain surgical procedures require more than simply cauterizing tissue and rely on the combination of clamping pressure, electrosurgical energy and gap distance to "seal" tissue, vessels and certain vascular bundles. More particularly, vessel sealing or tissue sealing is a recently-developed technology which utilizes a unique combination of radiofrequency energy, clamping pressure and precise control of gap distance (i.e., distance between opposing jaw members when closed about tissue) to effectively seal or fuse tissue between two opposing jaw members or sealing plates. Vessel or tissue sealing is more than "cauterization" which involves the use of heat to destroy tissue (also called "diathermy" or "electrodiathermy"). Vessel sealing is also more than "coagulation" which is the process of desiccating tissue wherein the tissue cells are ruptured and dried. "Vessel sealing" is defined as the process of liquefying the collagen, elastin and ground substances in the tissue so that the tissue reforms into a fused mass with significantly-reduced demarcation between the opposing tissue structures.

To effectively seal tissue or vessels, especially thick tissue and large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel or tissue being sealed. Accurate application of pressure is important for several reasons: to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal. It has been determined that the thickness of a typical fused vessel is optimum between about 0.03 and 0.2 mm (0.001 and about 0.006 inches). Below this range, the seal may shred or tear and above this range the tissue may not be properly or effectively sealed.

With respect to smaller vessels, the pressure applied becomes less relevant and the gap distance between the electrically conductive surfaces becomes more significant for effective sealing. In other words, the chances of the two electrically conductive surfaces touching during activation increases as the tissue thickness and the vessels become smaller.

Typically and particularly with respect to endoscopic electrosurgical procedures, once a vessel is sealed, the surgeon has to remove the sealing instrument from the operative site, substitute a new instrument through the cannula and accurately sever the vessel along the newly formed tissue seal. As can be appreciated, this additional step may be both time consuming (particularly when sealing a significant number of vessels) and may contribute to imprecise separation of the tissue along the sealing line due to the misalignment or misplacement of the severing instrument along the center of the tissue seal.

Sealing and electrically cutting on the same instrument is a recently developed technology which provides different advantages over mechanically cutting tissue. However, electrical cutting of tissue has proven difficult for manufacturing purposes due to the dimensions between the cutting electrodes being relatively small. Moreover, the sealing electrodes may produce heat formation and electrical charging during the seal cycle that may detrimentally affect the cut performance. For example, this tissue reaction may manifest during the seal cycle by damaging tissue within the cut zone and minimizing hydration by forcing conductive fluids from the intended cut area.

US 2004/0215185 discloses a working end for controlled energy delivery. The working end may have an elongate core of substantially resistive material for pre-heating the marking end.

Document US 2004/0254573 discloses a vessel sealer according to the preamble of claim 1. The invention is as described in the appended set of claims.

### SUMMARY

Document US 2004/0254573 discloses a vessel sealer according to the preamble of claim 1. The invention is as described in the appended set of claims.

The present invention provides an electrode assembly and a system for sealing tissue as defined by the claims.

The present disclosure relates to an electrode assembly for use with an instrument for sealing and/or cutting tissue. The electrode assembly includes a pair of opposing first and second jaw members at least one of which is movable relative to the other from a first position wherein the jaw members are disposed in spaced relation relative to one another to a second position wherein the jaw members cooperate to grasp tissue therebetween.

Each jaw member includes at least one electrically conductive tissue sealing surface extending along a length thereof, each tissue sealing surface being adapted to connect to a source of electrosurgical energy such that the tissue sealing surfaces are capable of conducting electrosurgical energy through tissue held therebetween to effect a seal. The jaw members each include an electrically conductive heating element disposed within the jaw members.

It is also disclosed a method for focusing energy to tissue and includes the initial step of providing a pair of opposing jaw members each having a pair of electrically conductive tissue sealing surfaces. At least one jaw member is movable relative to the other from a first position wherein the jaw members are disposed in spaced relation relative to one another to a second position wherein the jaw members cooperate to grasp tissue therebetween. Each jaw member includes an electrically conductive tissue sealing surface extending along a length thereof. The jaw members are adapted to connect to a source of electrosurgical energy such that the electrically conductive tissue sealing surfaces are capable of conducting electrosurgical energy through tissue held therebetween to effect a seal. The method further includes the step of pre-heating at least one electrically conductive tissue sealing surface before electrosurgical energy is applied.

The method further includes the step of positioning the opposing first and second jaw members about tissue and applying electrosurgical energy to said tissue. The method may also includes the step of directing electrosurgical energy to the at least one electrically conductive tissue sealing surfaces to cut and/or seal tissue.

The present invention also relates to a system for sealing tissue and that includes a pair of opposing first and second jaw members at least one of which is movable relative to the other from a first position wherein the jaw members are disposed in spaced relation relative to one another to a second position wherein the jaw members cooperate to grasp tissue therebetween. Each jaw member includes at least one electrically conductive tissue sealing surface that extends along a length thereof. The tissue sealing surfaces are adapted to connect to a source of electrosurgical energy such that the tissue sealing surfaces are capable of conducting electrosurgical energy through tissue held therebetween to effect a seal.

At least one heating element is included which is disposed within at least one of the jaw members. An electrical energy source operatively connects to each jaw member and provides electrical energy thereto.

### BRIEF DESCRIPTION OF THE DRAWINGS

Various embodiments of the subject instrument are described herein with reference to the drawings wherein:
FIG. 1 shows a right, perspective view of an endoscopic bipolar forceps having a housing, a shaft and a pair of jaw members affixed to a distal end thereof, the jaw members including an electrode assembly disposed therebetween;
FIG. 2 shows a left, perspective view of an open bipolar forceps showing a pair of first and second shafts each having a jaw member affixed to a distal end thereof with an electrode assembly disposed therebetween;
FIG. 3 shows a right, perspective view of an alternate embodiment of an open bipolar forceps showing a pair of first and second shafts each having a jaw member affixed to a distal end thereof with an electrode assembly disposed therebetween;
FIG. 4 shows an enlarged cross-sectional view of the vessel sealing instrument showing an electrode assembly having a heating element contained therein;
FIG. 5 shows an enlarged cross-sectional view of an alternate vessel sealing instrument showing an electrode assembly having a heating element contained therein;
FIG. 6 shows a side cross-sectional view of the vessel sealing instrument showing an electrode assembly having a heating element contained therein;
FIG. 7 shows a side cross-sectional view of an alternate vessel sealing instrument showing an electrode assembly having a heating element contained therein;
FIG. 8 shows a side view of the vessel sealing instrument showing an electrode assembly having a heating element contained therein;
FIG. 9 shows an exploded view of the vessel sealing instrument shown in FIG. 8;
FIG. 10 shows a side cross-sectional view of a vessel sealing instrument showing an electrode assembly having a heating element contained therein shown in an open configuration;
FIG. 11 shows a greatly-enlarged, side cross sectional view of the vessel sealing instrument of FIG. 10 showing an electrode assembly having a heating element contained therein;
FIG. 12 shows a greatly-enlarged, perspective view of an alternate top jaw of a vessel sealing instrument with parts separated; and
FIG. 13 shows an enlarged, side cross-sectional view of the vessel sealing instrument of FIG. 12 showing the jaw members in a spaced apart orientation.

### DETAILED DESCRIPTION

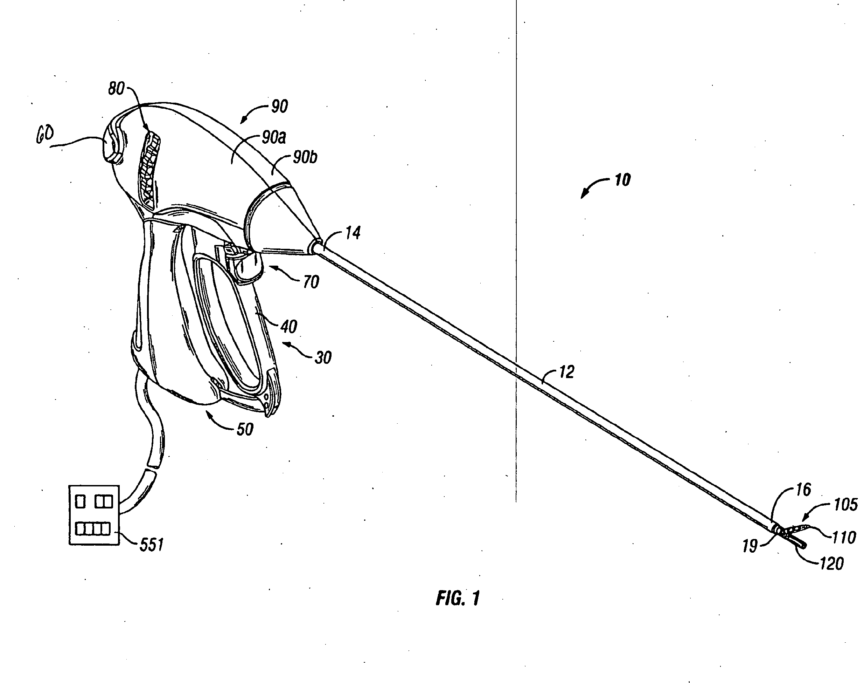

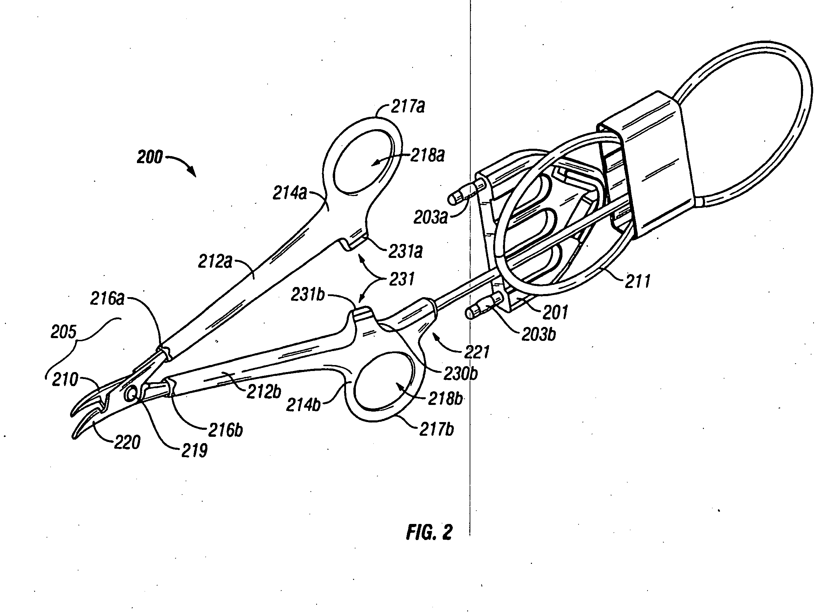

Referring now to the various figures, FIG. 1 depicts a bipolar forceps 10 for use in connection with endoscopic surgical procedures and FIG. 2 depicts an open forceps 200 contemplated for use in connection with traditional open surgical procedures. For the purposes herein, either an endoscopic instrument or an open instrument may be utilized with the various electrode assemblies described herein. Obviously, different electrical and mechanical connections and considerations apply to each particular type of instrument, however, the novel aspects with respect to the electrode assembly and its operating characteristics remain generally consistent with respect to both the open or endoscopic designs.

FIG. 1 shows a bipolar forceps 10 for use with various endoscopic surgical procedures and generally includes a housing 90, a handle assembly 30, a rotating assembly 80, a trigger assembly 70, switch assembly 60 and an electrode assembly 105 having opposing jaw members 110 and 120 which mutually cooperate to grasp, seal and divide tubular vessels and vascular tissue. More particularly, forceps 10 includes a shaft 12 which has a distal end 16 dimensioned to mechanically engage the electrode assembly 105 and a proximal end 14 which mechanically engages the housing 90. The shaft 12 may include one or more known mechanically engaging components which are designed to securely receive and engage the electrode assembly 105 such that the jaw members 110 and 120 are pivotable relative to one another to engage and grasp tissue therebetween.

The proximal end 14 of shaft 12 mechanically engages the rotating assembly 80 to facilitate rotation of the electrode assembly 105. In the drawings and in the descriptions which follow, the term "proximal", as is traditional, will refer to the end of the forceps 10 which is closer to the user, while the term "distal" will refer to the end which is further from the user. Details relating to one envisioned relationship among the various mechanically cooperating components of the shaft 12 and the rotating assembly 80 are described in commonly-owned U.S. Patent Application Serial No. 10/460,926 entitled "VESSEL SEALER AND DIVIDER FOR USE WITH SMALL TROCARS AND CANNULAS".

Handle assembly 30 includes a fixed handle 50 and a movable handle 40. Fixed handle 50 is integrally associated with housing 90 and handle 40 is movable relative to fixed handle 50 to actuate the opposing jaw members 110 and 120 of the electrode assembly 105 as explained in more detail below. Movable handle 40 and trigger assembly 70 are of unitary construction and are operatively connected to the housing 90 and the fixed handle 50 during the assembly process. Housing 90 is constructed from two components halves 90a and 90b which are assembled about the proximal end of shaft 12 during assembly. Trigger assembly 70 is configured to selectively provide electrical energy to the electrode assembly 105 for cutting tissue. Switch assembly 60 is disposed proximate to the rotating assembly 80 and is configured to selectively provide electrical energy to the jaw member 110 and 120 to effect a seal. It is envisioned that switch assembly 60 or trigger assembly 70 may be configured to activate both the sealing electrodes and the cutting electrodes during the tissue sealing and division cycle. The generator 551 may be configured to activate the various electrodes according to one or more algorithms. The electrodes may be activated sequentially or simultaneously depending upon a particular purpose.

As mentioned above, electrode assembly 105 is attached to the distal end 16 of shaft 12 and includes the opposing jaw members 110 and 120. Movable handle 40 of handle assembly 30 imparts movement of the jaw members 110 and 120 from an open position wherein the jaw members 110 and 120 are disposed in spaced relation relative to one another, to a clamping or closed position wherein the jaw members 110 and 120 cooperate to grasp tissue therebetween.

Referring now to FIG. 2, an open forceps 200 includes a pair of elongated shaft portions 212a and 212b each having a proximal end 214a and 214b, respectively, and a distal end 216a and 216b, respectively. The forceps 200 includes jaw members 210 and 220 which attach to distal ends 216a and 216b of shafts 212a and 212b, respectively. The jaw members 210 and 220 are connected about pivot pin 219 which allows the jaw members 210 and 220 to pivot relative to one another from the first to second positions for treating tissue. The electrode assembly 205 is connected to opposing jaw members 210 and 220 and may include electrical connections through or around the pivot pin 219. Examples of various electrical connections to the jaw members are shown in commonly-owned U.S. Patent Application Serial Nos. 10/474,170, 10/116,834, 10/284,562 10/472,295, 10/116,944, 10/179,863 and 10/369,894.

Each shaft 212a and 212b includes a handle 217a and 217b disposed at the proximal end 214a and 214b thereof which each define a finger hole 218a and 218b, respectively, therethrough for receiving a finger of the user. As can be appreciated, finger holes 218a and 218b facilitate movement of the shafts 212a and 212b relative to one another which, in turn, pivot the jaw members 210 and 220 from the open position wherein the jaw members 210 and 220 are disposed in spaced relation relative to one another to the clamping or closed position wherein the jaw members 210 and 220 cooperate to grasp tissue therebetween. A ratchet 231 is included for selectively locking the jaw members 210 and 220 relative to one another at various positions during pivoting.

More particularly, the ratchet 231 includes a first mechanical interface 231 a associated with shaft 212a and a second mating mechanical interface 231 b associated with shaft 212b. Each position associated with the cooperating ratchet interfaces 231 a and 231 b holds a specific, i.e., constant, strain energy in the shaft members 212a and 212b which, in turn, transmits a specific closing force to the jaw members 210 and 220. It is envisioned that the ratchet 231 may include graduations or other visual markings which enable the user to easily and quickly ascertain and control the amount of closure force desired between the jaw members 210 and 220.

As best seen in FIG. 2, forceps 200 may also include an electrical interface or plug 201 which connects the forceps 200 to a source of electrosurgical energy, e.g., an electrosurgical generator 551 (See FIG. 1). Plug 201 includes at least two prong members 203a and 203b which are dimensioned to mechanically and electrically connect the forceps 200 to the electrosurgical generator 551. An electrical cable 211 extends from the plug 201 and securely connects the cable 211 to the forceps 200. Cable 211 is internally divided within the shaft 212b to transmit electrosurgical energy through various electrical feed paths to the electrode assembly 205.

One of the shafts, e.g., 212b, includes a proximal shaft connector/flange 221 which is designed to connect the forceps 200 to the electrosurgical generator 551. More particularly, flange 221 mechanically secures electrosurgical cable 211 to the forceps 200 such that the user may selectively apply electrosurgical energy as needed.

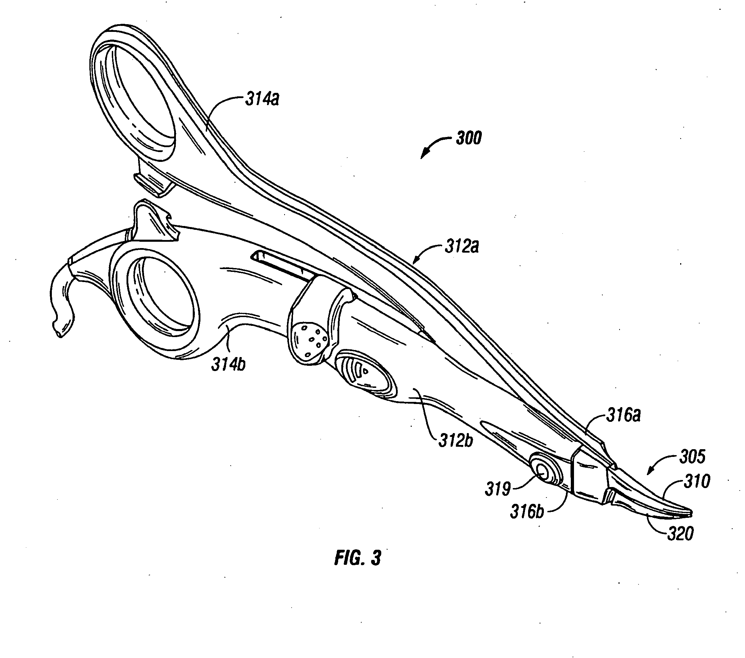

Referring now to FIG. 3, another open forceps 300 is shown for use with open surgical procedures includes elongated shaft portions 312a and 312b each having a proximal end 314a, 314b and a distal end 316a and 316b, respectively. Forceps 300 includes an electrode assembly 305 which attaches to the distal ends 316a and 316b of shafts 312a and 312b, respectively. As explained in more detail below, the electrode assembly 305 includes pair of opposing jaw members 310 and 320 which are pivotably connected about a pivot pin 319 and which are movable relative to one another to grasp tissue.

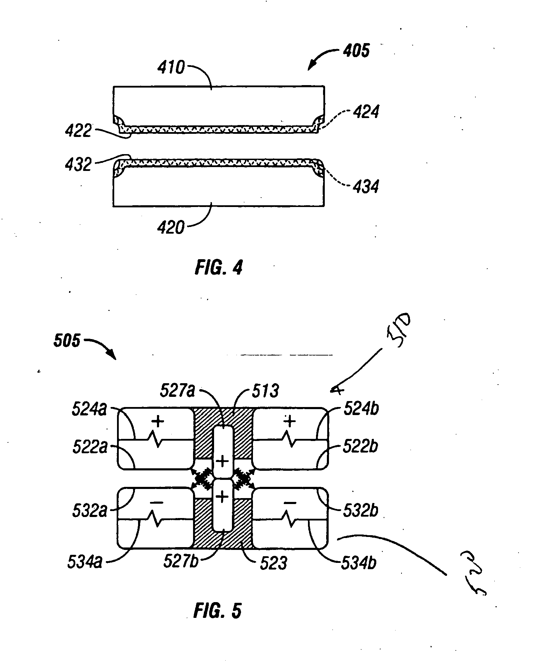

Referring now to FIG. 4, a cross-sectional view of electrode assembly 405 is shown. Electrode assembly 405 includes first and second opposing jaw members 410 and 420. Jaw members 410 and 420 include electrically conductive tissue sealing surfaces 422 and 432. Tissue sealing surfaces 422 and 432 are adapted to connect to a source of electrosurgical energy (i.e., generator 551) and are capable of conducting electrosurgical energy through tissue held therebetween to effect a seal. As shown in FIG. 4, tissue sealing surfaces 422 and 432 further include electrically conductive heating elements 424 and 434 which are embedded within tissue sealing surfaces 422 and 432. Heating elements 424 and 434 are configured to pre-heat electrically conductive tissue sealing surfaces 422 and 432 before electrosurgical energy is applied to the tissue. This allows for an increased electrode mass, more stable temperature profiles and more predictable seals. Heating elements 424 and 434 may be constructed from a variety of different materials including, but not limited to, Nichrome wire, Nichrome ribbon, Calrod and PTC ceramics.

FIG. 5 discloses yet another embodiment according to the present disclosure and includes sealing surfaces 522a, 522b and 532a, 532b, insulators 513 and 523 and cutting elements 527a and 527b. Insulators 513 and 523 could be constructed of a variety of different materials, including, but not limited to, glass, ceramic and polymeric materials. With this particular embodiment, during the cutting phase, both sets of opposing sealing surfaces 522a, 532a and 522b, 532b are energized with the second electrical potential "-" and the cutting elements 527a and 527b are energized to the first electrical potential "+". It is believed that this electrode assembly 505 will create concentrated electrical paths between the potentials "+" and "-" through the tissue to cut the tissue between the previously formed tissue seals. Heating elements 524a, 524b and 534a, 534b are shown disposed within tissue sealing surfaces 522a, 532a and 522b, 532b and act to pre-heat the sealing surfaces 522a, 532a and 522b, 532b. It is envisioned that additional heating elements may be included within cutting elements 527a and 527b to pre-heat the cutting electrodes prior to activation.

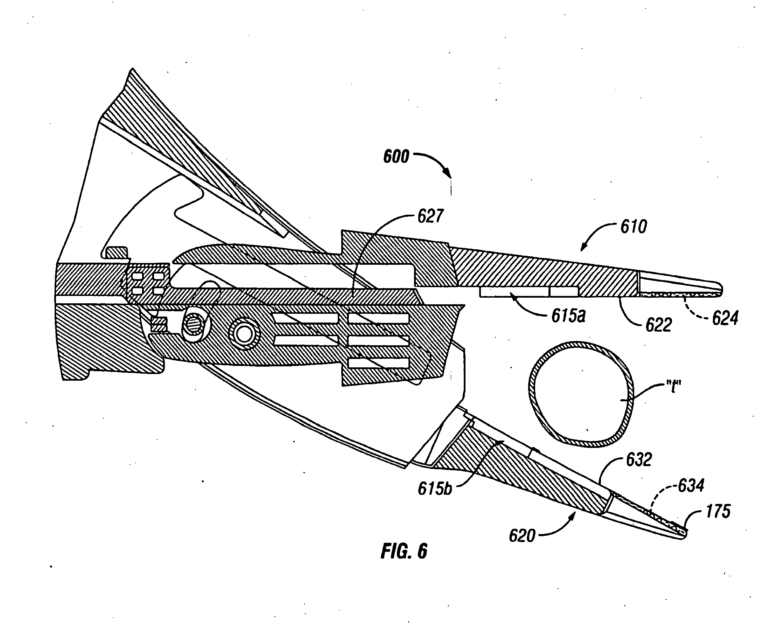

Referring now to FIG. 6, an alternative embodiment of a forceps 600 according to the present disclosure is shown. Forceps 600 includes first and second opposing jaw members 610 and 620 having tissue sealing surfaces 622 and 632 disposed thereon. Heating elements 624 and 634 are contained within tissue sealing surfaces 622 and 632 as described above. Jaw member 620 includes a knife channel 615a disposed within the sealing surface 632 which allows a knife 627 to translate through the jaw member 620. Jaw member 610 may also include a knife channel 615b disposed in vertical registration with knife channel 615a. When jaw members 610 and 620 are in the closed position, cutting element 627 is selectively extendable through knife channels 615a and 615b to cut tissue "t" disposed between jaw members 610 and 620.

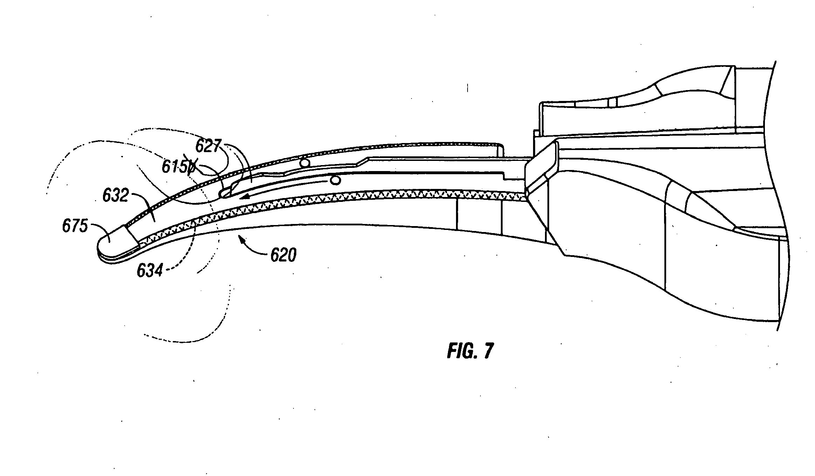

FIG. 7 shows a top perspective view of second jaw member 620. Jaw member 620 includes tissue sealing surface 632 having heating element 634 disposed therein. As mentioned above, additional heating elements are disposed within cutting element 627 according to the present invention. It is envisioned that one of the jaw members, e.g., 620, may include at least one stop member 675 disposed on the inner facing surface of the electrically conductive sealing surface 632 (and/or 622). A more detailed description of this embodiment is described in commonly-owned App. Ser. No. 10/962, 116.

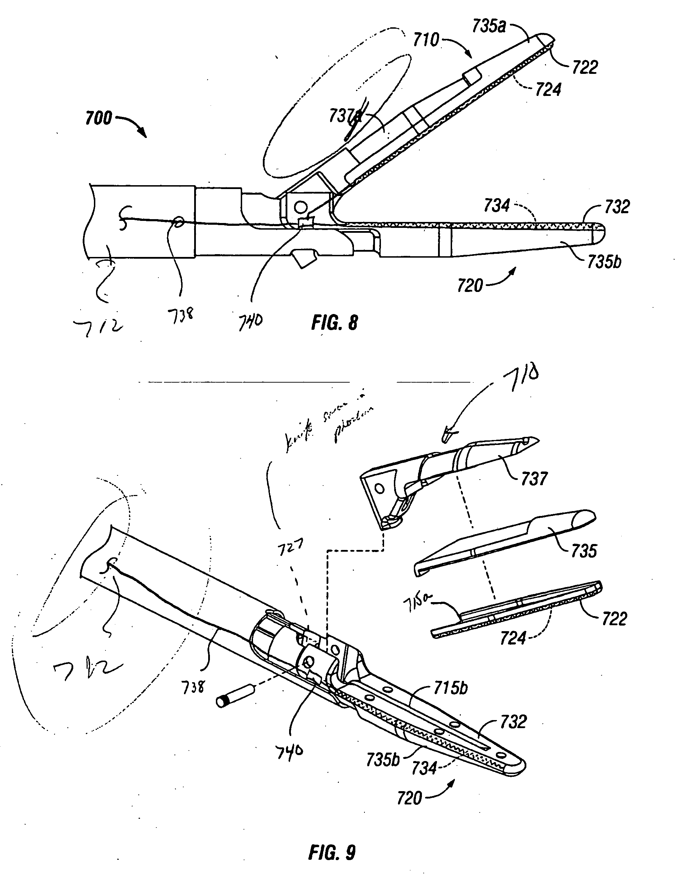

Referring now to FIGS. 8-9, an alternative endoscopic forceps 700 is shown. Forceps 700 includes jaw members 710, 720 having electrically conductive tissue sealing surfaces 722, 732 which are substantially surrounded by an insulating substrate 735a and 735b. Forceps 700 is designed as a unilateral jaw assembly in which only one jaw member moves relative to the other jaw member 720. Insulator 735a and 735b, electrically conductive sealing surfaces 722 and 732 and outer, non-conductive jaw housing 737 are dimensioned to limit and/or reduce many of the known undesirable effects related to tissue sealing, e.g., flashover, thermal spread and stray current dissipation. It is also envisioned that the jaw members 710 and 720 may be manufactured from a ceramic-like material and the electrically conductive surface(s) 722 may be coated onto the ceramic-like jaw members 710 and 720. Heating elements 724 and 734 may be contained within tissue sealing surfaces 722 and 732 as described above.

As best illustrated in FIG. 9, a knife channel is utilized to guide the cutting element 727 along a predefined cutting path. Knife channel 715 (composed of top and bottom channels 715a and 715b) runs through the center of the jaw members 710 and 720, respectively, such that a cutting element 727 (shown in phantom) can cut the tissue "t" grasped between the jaw members 710 and 720 when the jaw members 710 and 720 are in a closed position. Cutting element 727 can only be advanced through the tissue "t" when the jaw members 710 and 720 are closed thus preventing accidental or premature activation of the cutting element 727 through the tissue "t". Heating elements 724 and 734 may be contained within tissue sealing surfaces 722 and 732 and are precontained within cutting element 727 according to the invention.

It is envisioned that separate electrical connections (e.g., 738) may be utilized to connect the heating elements 724 and 734 to generator 551 or an alternate energy source (not shown). Alternatively, the same electrical connections may be employed to energize both the sealing surfaces 722 and 732 and the heating elements 724 and 734 or the cutting electrodes (See FIG. 5) and the heating elements 724 and 734. Moreover, it is envisioned that a switching mechanism 740 may be included in one or more jaw members 710 and 720 (or 610 and 620, 510 and 520, 410 and 420, 310 and 320, 210 and 220 or 110 and 120) or one or more shafts 712 (or 212a, 212b or 12) which switches between the varying heat sources based upon generator control.

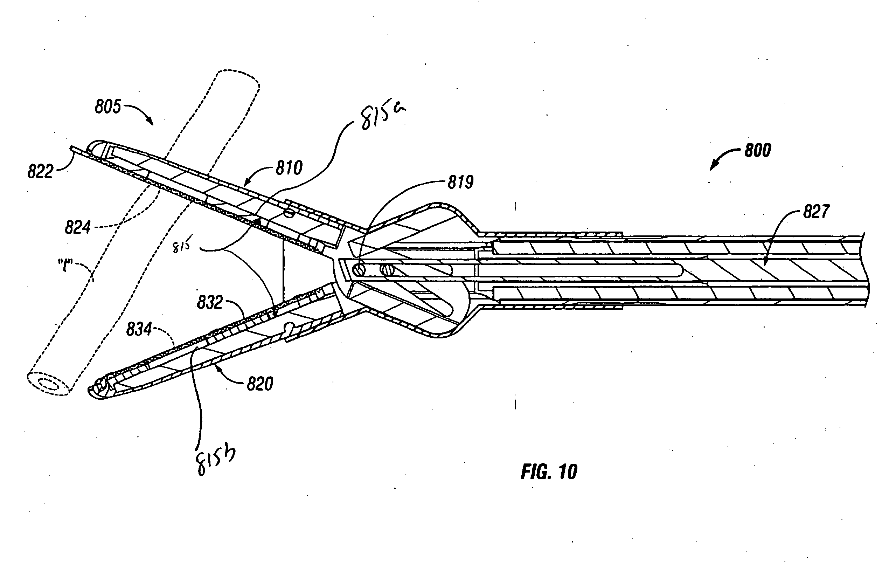

Referring now to FIGS. 10-11, an alternative forceps 800 according to the present disclosure is shown. Electrode assembly 805 includes opposing jaw members 810 and 820 which cooperate to effectively grasp tissue for sealing purposes. The electrode assembly 805 is designed as a bilateral assembly, i.e., both jaw members 810 and 820 pivot relative to one another about a pivot pin 819 disposed therethrough. In this arrangement, heating elements 824 and 834 are contained within tissue sealing surfaces 822 and 832 similar to those embodiments described above. Knife channel 815a is configured to receive cutting element 827 as above. Heating elements are energized via one or more electrical connection 838 which may be regulated by generator 551 or another heat source in a similar manner as described above.

Knife channel 815 is formed when the jaw members 810 and 820 are closed and the two knife channels 815a and 815b align. Knife channel 815 may be configured as a straight slot with no degree of curvature which, in turn, causes the cutting element 827 to move through the tissue in a substantially straight fashion. Alternatively, the knife channel 815 may be dimensioned to include some degree of curvature to cause the cutting element 827 to move through tissue "t" in a curved fashion. Insulating plate 839 also forms part of the knife channel 815 and includes a channel 815a' defined therein which extends along insulating plate 839 and which aligns in vertical registration with knife channel half 815a to facilitate translation of cutting element 827 therethrough. A more detailed description of this embodiment is described in commonly-owned U.S. Prov. App. No. 60/722,177 filed 9/30/05.

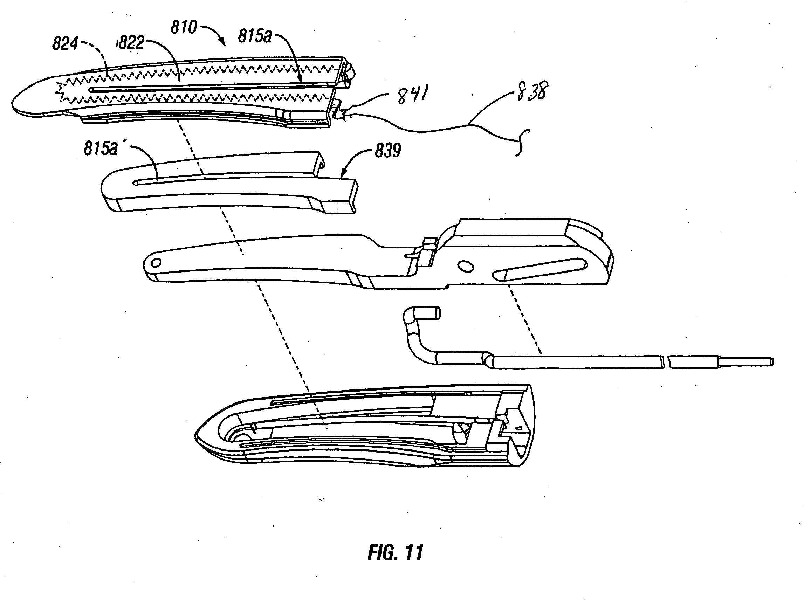

As best seen in FIG. 11, the heating element 824 is configured to heat the sealing surface 822 upon activation thereof. Heating element 824 may be configured to wrap around the knife channel as shown. One or more electrical crimp-like connectors 841 may be employed to connect the electrical connections to the heating element 824.

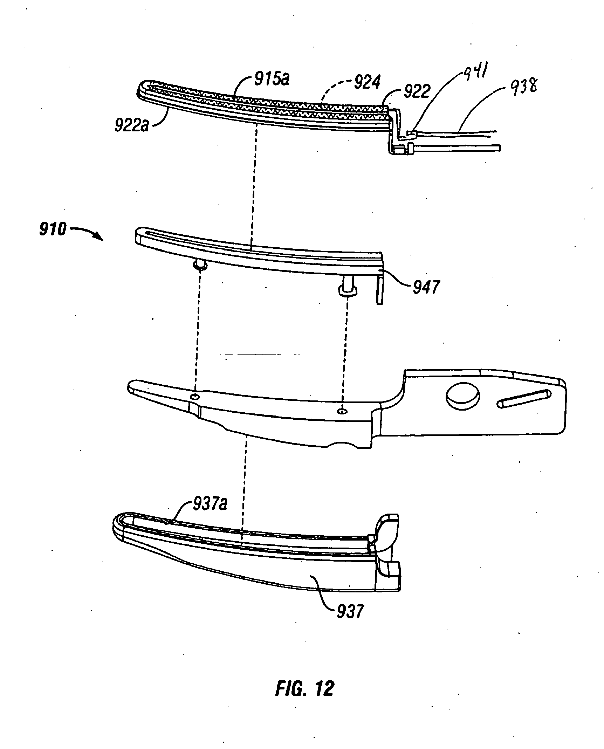

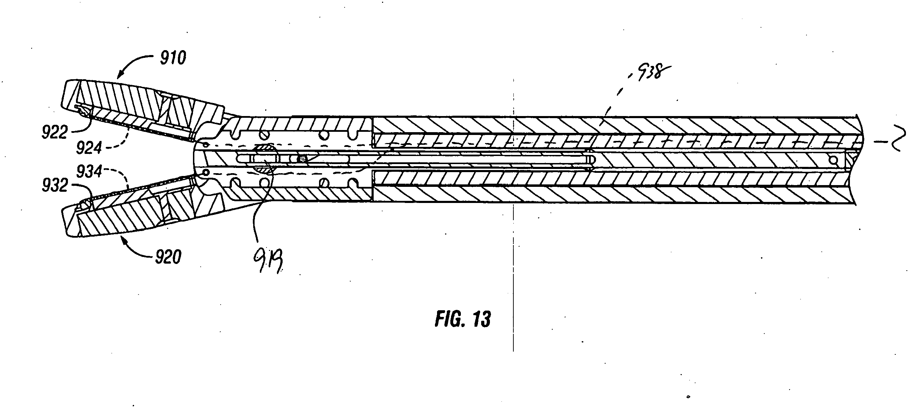

Referring now to FIGS. 12-13, another embodiment of an electrode assembly 905 is shown which includes opposing jaw members 910 and 920 which cooperate to effectively grasp tissue for sealing purposes. The-electrode assembly 905 is designed as a bilateral assembly, i.e., both jaw members 910 and 920 pivot relative to one another about a pivot pin 919 disposed therethrough. The jaw members 910 and 920 are curved to facilitate manipulation of tissue and to provide better "line of sight" for accessing organs and large tissue structures. Electrically conductive sealing plate 922 includes a peripheral flange 922a which surrounds the periphery of the sealing plate 922. Flange 922a is designed to matingly engage an inner lip 937a of the outer insulator 937. This may be accomplished by known processes, e.g., overmolding. Heating elements 924 and 934 are disposed within electrically conductive tissue sealing surfaces 922 and 932 respectively. Insulator 947, electrically conductive sealing surface 922 and the outer, non-conductive jaw housing 937 are dimensioned to limit and/or reduce many of the known undesirable effects related to tissue sealing, e.g., flashover, thermal spread and stray current dissipation. As best seen in FIG. 12, a crimp connector 941 may be used to connect the heating element 924 to the energy source via cable or electrical connection 938. Electrical connection 938 is routed through shaft 912 and through the instrument to electrically connect to an energy source.

It is envisioned that the electrically conductive heating elements may be activated prior to the application of electrosurgical energy, in conjunction with the application of electrosurgical energy or after the application of electrosurgical energy. Moreover, it is within the scope of the present disclosure for the electrically conductive heating elements to be disposed within one or both of the jaw members and/or within the electrically conductive cutting element.

As can be appreciated, the various geometrical configurations and electrical arrangements of the aforementioned electrode assemblies allow the surgeon to initially activate the two opposing electrically conductive tissue contacting surfaces and seal the tissue and, subsequently, selectively and independently divide tissue either mechanically with a translatable knife or electrically by activating the cutting element and one or more tissue contacting surfaces to cut the tissue utilizing the various above-described and shown electrode assembly configurations. Hence, the tissue is initially sealed and thereafter cut without re-grasping the tissue.

However, it is envisioned that the cutting element may be deployed mechanically without sealing or energized with one or more tissue contacting surfaces to simply cut tissue/vessels without initially sealing. For example, the jaw members may be positioned about tissue and the cutting element may be selectively activated to separate or simply coagulate tissue. This type of alternative embodiment may be particularly useful during certain endoscopic procedures wherein an electrosurgical pencil is typically introduced to coagulate and/or dissect tissue during the operating procedure.

Trigger 70 may be employed to allow the surgeon to selectively activate (energize) one or more tissue contacting surfaces, the pre-heating elements and/or the cutting element to cut tissue. As can be appreciated, this allows the surgeon to initially seal tissue and then activate the cutting element by simply activating the trigger. Alternatively, the same switch may be employed to initially seal tissue and then cut tissue after a successful seal has been confirmed by the generator algorithm, then the surgeon grasps the tissue and activates one switch to seal and divide tissue.

One or more switches can be placed anywhere on the instrument or may be configured as a remote switch, e.g., handswitch or footswitch. It is also envisioned that the switch may cooperate with a smart sensor (or smart circuit, computer, feedback loop, etc.) which automatically triggers the switch to change between the "pre-heating mode", the "sealing" mode and the "cutting" mode upon the satisfaction of a particular parameter. For example, the smart sensor may include a feedback loop which indicates when a tissue seal is complete based upon one or more of the following parameters: tissue temperature, tissue impedance at the seal, tissue type, hydration, change in impedance of the tissue over time and/or changes in the power or current applied to the tissue over time. An audible or visual feedback monitor may be employed to convey information to the surgeon regarding the overall seal quality or the completion of an effective tissue seal. A separate lead may be connected between the smart sensor and the generator for visual and/or audible feedback purposes. The smart sensor may be disposed in a variety of different arrangements, including, but not limited to, within or upon the electrically conductive tissue sealing surfaces, cutting elements, or anywhere therebetween.

In one embodiment, the generator 551 delivers energy to the tissue in a pulse-like waveform. Delivering the energy in pulses may increase the amount of sealing energy which can be effectively delivered to the tissue and reduce unwanted tissue effects such as charring. Moreover, the feedback loop of the smart sensor can be configured to automatically measure various tissue parameters during sealing (i.e., tissue temperature, tissue impedance, hydration, heating rate, tissue type, current through the tissue) and automatically adjust the energy intensity and number of pulses as needed to reduce various tissue effects such as charring and thermal spread.

It is also envisioned that electrode assembly 105 (or 205, 305, etc.) may include a sensor capable of detecting temperature changes or changes in the heating rate of the electrode. The sensor may be contained within electrode 105 and could communicate with a feedback control mechanism housed within electrosurgical generator 551 or elsewhere. Various forms of feedback control are well-known and may be utilized in the present disclosure. For a detailed description of modern feedback control systems see FEEDBACK CONTROL OF DYNAMIC SYSTEMS, by G. Franklin et al., Prentice-Hall, Upper Saddle River, NJ, 2002.

It has also been determined that RF pulsing may be used to more effectively cut tissue. For example, an initial pulse from the cutting element through the tissue (or the tissue contacting surfaces through the tissue) may be delivered to provide feedback to the smart sensor for selection of the ideal number of subsequent pulses and subsequent pulse intensity to effectively and consistently cut the amount or type of tissue with minimal effect on the tissue seal. If the energy is not pulsed, the tissue may not initially cut but desiccate since tissue impedance remains high during the initial stages of cutting. By providing the energy in short, high energy pulses, it has been found that the tissue is more likely to cut.

Alternatively, a switch may be configured to activate based upon a desired cutting parameter and/or after an effective seal is created or has been verified. For example, after effectively sealing the tissue, the cutting element may be automatically activated based upon a desired end tissue thickness at the seal.

As mentioned in many of the above embodiments, upon compression of the tissue, the cutting element may act as a stop member and create a gap "G" between the opposing conductive tissue contacting surfaces. Particularly with respect to vessel sealing, the gap distance is in the range of about 0.03 mm and 0.2 mm (0.001 to about 0.006 inches). As mentioned above, controlling both the gap distance "G" and clamping pressure between conductive surfaces are two important mechanical parameters which need to be properly controlled to assure a consistent and effective tissue seal. The surgeon activates the generator to transmit electrosurgical energy to the tissue contacting surfaces and through the tissue to effect a seal. As a result of the unique combination of the clamping pressure, gap distance "G" and electrosurgical energy, the tissue collagen melts into a fused mass with limited demarcation between opposing vessel walls.

Once pre-heated and sealed, the surgeon advances a knife or activates the cutting element to cut the tissue. As mentioned above, the surgeon does not necessarily need to re-grasp the tissue to cut, i.e., the cutting element is already positioned proximate the ideal, center cutting line of the seal. During an electrical cutting phase, highly concentrated electrosurgical energy travels from the cutting element through the tissue to cut the tissue into two distinct halves. As mentioned above, the number of pulses required to effectively cut the tissue and the intensity of the cutting energy may be determined by measuring the seal thickness and/or tissue impedance and/or based upon an initial calibrating energy pulse which measures similar parameters. A smart sensor (not shown) or feedback loop may be employed for this purpose.

As can be appreciated, the forceps may be configured to automatically cut the tissue once sealed or the instrument may be configured to permit the surgeon to selectively divide the tissue once sealed. Moreover, it is envisioned that an audible or visual indicator (not shown) may be triggered by a sensor (not shown) to alert the surgeon when an effective seal has been created. The sensor may, for example, determine if a seal is complete by measuring one of tissue impedance, tissue opaqueness and/or tissue temperature. Commonly-owned U.S. Application Serial No. 10/427,832 several electrical systems which may be employed to provide positive feedback to the surgeon to determine tissue parameters during and after sealing and to determine the overall effectiveness of the tissue seal.

It is envisioned that heating elements 424 and 434 (or any of the other envisioned heating elements 524, 534, 624, 634, 724, 734, 824, 834, 924 and/or 934 as shown and described herein) may be configured in a variety of different arrangements. Heating elements 424 and 434 are fixed resistors, variable resistors, varistors, sensitors or thermistors. Elements 424 and 434 may be located in a variety of different areas within electrode assembly 405. Moreover, it is envisioned that the heating elements 424 and 434 may be fixed resistive layers, such as those used during silicon wafer fabrication.

In operation, jaw members 110 and 120 (or 210 and 220, 310 and 320, 410 and 420, etc.) each having a pair of electrically conductive tissue sealing surfaces 422 and 432 (or 522 and 532, etc.) are provided. One jaw member (either 110 or 120, or 210 and 220, etc.) is movable relative to the other from a first position wherein the jaw members are disposed in spaced relation relative to one another to a second position wherein the jaw members cooperate to grasp tissue "t" therebetween (See FIGS. 6 and 10). Each jaw member (either 110 or 120, or 210 and 220, etc.) is adapted to connect to a source of electrosurgical energy (such as electrosurgical generator 551) such that the electrically conductive tissue sealing surfaces 422 and 432 (or 522 and 532, etc.) are capable of conducting electrosurgical energy through tissue "t" held therebetween to effect a seal. The electrically conductive tissue sealing surfaces 422 and 432 (or 522 and 532, etc.) include heating elements 424 and 434 (or 524 and 534, etc.) disposed therein. Electrically conductive heating elements 424 and 434 (or 524 and 534, etc.) are configured to pre-heat at least one electrically conductive tissue sealing surface 422 and 432 (or 522 and 532, etc.) before electrosurgical energy is applied.

Opposing jaw members (either 110 or 120, or 210 and 220, etc.) are positioned about tissue "t" and electrosurgical energy is applied from electrosurgical generator 551 to the electrically conductive heating elements 424 and 434 (or 524 and 534, etc.) to pre-heat at least one electrically conductive tissue sealing surface 422 and 432. Once sealing surfaces 422 and 432(or 522 and 532, etc.) are pre-heated electrosurgical energy from electrosurgical generator 551 is directed to electrically conductive tissue sealing surfaces 422 and 432 (or 522 and 532, etc.) to cut and/or seal tissue "t".

The electrosurgical intensity from each of the electrically conductive surfaces, the pre-heating elements and cutting elements may be selectively or automatically controllable to assure consistent and accurate cutting along the centerline of the tissue in view of the inherent variations in tissue type and/or tissue thickness. Moreover, it is contemplated that the entire surgical process may be automatically controlled such that after the tissue is initially grasped the surgeon may simply activate the forceps to seal and subsequently cut tissue. In this instance, the generator may be configured to communicate with one or more sensors (not shown) to provide positive feedback to the generator during the pre-heat, sealing and cutting processes to insure accurate and consistent sealing and division of tissue. Commonly-owned U.S. Patent Application Serial No. 10/427,832 discloses a variety of feedback mechanisms which may be employed for this purpose.

From the foregoing and with reference to the various figure drawings, those skilled in the art will appreciate that certain modifications can also be made to the present disclosure without departing from the scope of the present disclosure. For example, it is contemplated that cutting element may be dimensioned as a cutting wire which is selectively activatable by the surgeon to divide the tissue after sealing. More particularly, a wire is mounted within the insulator between the jaw members and is selectively energizable upon activation of the switch.

The forceps may be designed such that it is fully or partially disposable depending upon a particular purpose or to achieve a particular result. For example, the electrode assembly may be selectively and releasably engageable with the distal end of the shaft and/or the proximal end of shaft may be selectively and releasably engageable with the housing and the handle assembly. In either of these two instances, the forceps would be considered "partially disposable" or "reposable", i.e., a new or different electrode assembly (or electrode assembly and shaft) selectively replaces the old electrode assembly as needed.

It is envisioned that the electrode assembly could be selectively detachable (i.e., reposable) from the shaft depending upon a particular purpose, e.g., it is contemplated that specific forceps could be configured for different tissue types or thicknesses. Moreover, it is envisioned that a reusable forceps could be sold as a kit having different electrodes assemblies for different tissue types. The surgeon simply selects the appropriate electrode assembly for a particular tissue type.

It is also envisioned that the forceps could include a mechanical or electrical lockout mechanism which prevents the pre-heating element(s), sealing surfaces and/or the cutting element(s) from being unintentionally activated when the jaw members are disposed in the open configuration.

Although the subject forceps and electrode assemblies have been described with respect to particular embodiments, it will be readily apparent to those having ordinary skill in the art to which it appertains that changes and modifications may be made thereto. For example, although the specification and drawings disclose that the electrically conductive surfaces may be employed to initially seal tissue prior to electrically cutting tissue in one of the many ways described herein, it is also envisioned that the electrically conductive surfaces may be configured and electrically designed to perform any known bipolar or monopolar function such as electrocautery, hemostasis, and/or desiccation utilizing one or both jaw members to treat the tissue. Moreover, the jaw members in their presently described and illustrated formation may be energized to simply cut tissue without initially sealing tissue which may prove beneficial during particular surgical procedures. Moreover, it is contemplated that the various geometries of the jaw members, cutting elements, insulators and semi-conductive materials and the various electrical configurations associated therewith may be utilized for other surgical instrumentation depending upon a Document US 2004/0254573 discloses a vessel sealer according to the preamble of claim 1. The invention is as described in the appended set of claims. particular purpose, e.g., cutting instruments, coagulation instruments, electrosurgical scissors, etc.

It is envisioned that electrosurgical generator 551 may supply energy from a variety of different sources having a range of operating frequencies. Some of these include, but are not limited to, RF energy, microwave, infrared, ultraviolet, X-Ray, and ultrasonic (e.g., an ultrasonic heater capable of providing sonic waves into tissue). Moreover, electrosurgical generator 551 may supply either alternating or direct current and may produce continuous or pulse-like waveforms of varying periodicity, frequency and wavelengths.

It is also envisioned that the geometry of the electrodes may be configured such that the surface area ratios between the electrical poles focus electrical energy at the tissue. Moreover, it is envisioned that the geometrical configurations of the electrodes and insulators may be designed such that they act like electrical sinks or insulators to influence the heat effect within and around the tissue during the sealing or cutting processes.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope of the claims appended hereto.

## Claims

1. An electrode assembly (700) comprising:
a pair of opposing first and second jaw members (620, 710, 720) at least one of which is movable relative to the other from a first position wherein the jaw members are disposed in spaced relation relative to one another to a second position wherein the jaw members cooperate to grasp tissue therebetween;
each jaw member including at least one electrically conductive tissue sealing surface (632, 732, 722) extending along a length thereof, each tissue sealing surface being adapted to connect to a source of electrosurgical energy such that the at least one electrically conductive tissue sealing surfaces are capable of conducting electrosurgical energy through tissue held therebetween to effect a seal;
at least one first heating element (634, 734, 724) disposed within at least one of the jaw members, the at least one first heating element configured to pre-heat the at least one electrically conducive tissue sealing surface before electrosurgical energy is applied;
a knife (627);
one or both of said jaw members including a knife channel (615, 715) utilized to guide the knife along a predefined cutting path to cut tissue grasped between the jaw members when the jaw members are in a closed position; and **characterised by**:
at least one second heating element contained within the knife, said second heating element being a fixed resistor, variable resistor, sensitor or thermistor.

2. The electrode assembly according to claim 1, wherein the first or the second heating element is selected from the group consisting of Nichrome wire, Nichrome ribbon, Calrod and PTC ceramic.

3. The electrode assembly according to claim 1 or 2, further comprising an insulative material disposed on each jaw member, the insulative material selected from the group consisting of glass, ceramic and polymeric materials.

4. The electrode assembly according to claim 1, 2 or 3, further comprising at least one sensor configured to measure the temperature of the at least one electrically conductive tissue sealing surface.

5. The electrode assembly according to claim 1, 2, 3 or 4, further comprising at least one sensor configured to measure the temperature of the tissue.

6. A system for sealing tissue comprising:
an electrode assembly according to any one of the preceding claims; and
a source (551) of electrical energy operatively connected to each jaw member, the source of electrical energy configured to provide electrical energy thereto.

7. The system according to claim 6 further comprising a feedback control mechanism operable to predict a heating rate.

8. The system according to claim 6 or 7 further comprising at least one sensor configured to measure the temperature of the at least one electrically conductive tissue sealing surface.

## Patentansprüche

1. Elektrodenaufbau (700), mit:
einem Paar gegenüberstehender erster und zweiter Backenelemente (620, 710, 720), von denen mindestens eines relativ zu dem anderen von einer ersten Position, in der die Backenelemente relativ zueinander in beabstandeter Beziehung sind, zu einer zweiten Position, in der die Backenelemente zusammenwirken, um zwischen ihnen Gewebe zu greifen, bewegbar ist;
wobei jedes Backenelement mindestens eine elektrisch leitfähige gewebeversiegelnde Fläche (632, 732, 722) aufweist, die sich entlang einer Länge davon erstreckt, wobei jede gewebeversiegelnde Fläche angepasst ist, sich so mit einer Quelle elektrochirurgischer Energie zu verbinden, dass mindestens eine elektrisch leitfähige gewebeversiegelnde Fläche imstande ist, elektrochirurgische Energie durch dazwischen gehaltenes Gewebe zu leiten, um eine Versiegelung zu bewirken;
mindestens einem ersten Heizelement (634, 734, 724), das zumindest in einem der Backenelemente angeordnet ist, wobei das mindestens eine Heizelement eingerichtet ist, die mindestens eine elektrisch leitfähige gewebeversiegelnde Fläche bevor elektrochirurgische Energie angewandt wird vorzuwärmen;
einem Messer (627);
wobei eines oder beide der Backenelemente einen Messerkanal (615, 715) aufweist, der verwendet wird, das Messer entlang eines vorbestimmten Schneidpfads zu führen, um zwischen den Backenelementen ergriffenes Gewebe zu schneiden, wenn die Backenelemente in einer geschlossenen Position sind; und **gekennzeichnet durch**
mindestens ein zweites Heizelement, das in dem Messer aufgenommen ist, wobei das zweite Heizelement ein konstanter Widerstand, ein veränderlicher Widerstand, ein Sensistor oder ein Heißleiter ist.

2. Elektrodenaufbau nach Anspruch 1, bei dem das erste oder zweite Heizelement aus der Gruppe, die aus Nichromdraht, Nichromband, Calrod und PTC-Keramik besteht, ausgewählt ist.

3. Elektrodenaufbau nach Anspruch 1 oder 2, des Weiteren mit einem isolierenden Material, das auf jedem Backenelement angeordnet ist, wobei das isolierende Material aus der Gruppe, die aus Glas, Keramik und Polymermaterialien besteht, ausgewählt ist.

4. Elektrodenaufbau nach Anspruch 1, 2 oder 3, des Weiteren mit mindestens einem Sensor, der eingerichtet ist, die Temperatur von der mindestens einen elektrisch leitfähigen gewebeversiegelnden Fläche zu messen.

5. Elektrodenaufbau nach Anspruch 1, 2, 3 oder 4, des Weiteren mit mindestens einem Sensor, der eingerichtet ist, die Temperatur des Gewebes zu messen.

6. System zum Versiegeln von Gewebe, mit:
einem Elektrodenaufbau nach einem der vorstehenden Ansprüche; und
einer Quelle (551) elektrochirurgischer Energie, die betriebsfähig mit jedem Backenelement verbunden ist, wobei die Quelle elektrochirurgischer Energie eingerichtet ist, diesem elektrische Energie zuzuführen.

7. System nach Anspruch 6, des Weiteren mit einem Feedback-Regelmechanismus, um im Betrieb eine Heizgeschwindigkeit zu regeln.

8. System nach Anspruch 6 oder 7, des Weiteren mit mindestens einem Sensor, der eingerichtet ist, die Temperatur von der mindestens einen elektrisch leitfähigen gewebeversiegelnden Fläche zu messen.

## Revendications

1. Ensemble d'électrodes (700) comprenant :
une paire de premier et deuxième éléments de mâchoire opposés (620, 710, 720) dont au moins un est déplaçable relativement à l'autre d'une première position dans laquelle les éléments de mâchoire sont disposés en une relation espacée l'un relativement à l'autre à une seconde position dans laquelle les éléments de mâchoire coopèrent pour saisir le tissu entre eux ;
chaque élément de mâchoire incluant au moins une surface d'obturation de tissu électriquement conductrice (632, 732, 722) s'étendant sur une longueur de celui-ci, chaque surface d'obturation de tissu étant apte à être connectée à une source d'énergie électro-chirurgicale de sorte que la au moins une des surfaces d'obturation de tissu électriquement conductrices sont aptes à conduire l'énergie électro-chirurgicale à travers le tissu tenu entre elles pour effectuer l'obturation ;
au moins un premier élément chauffant (634, 734, 724) disposé dans au moins un des éléments de mâchoire, le au moins un premier élément chauffant étant configuré pour préchauffer la au moins une surface d'obturation de tissu électriquement conductrice avant l'application de l'énergie électro-chirurgicale ;
un couteau (627) ;
un ou les deux desdits éléments de mâchoire incluant un canal de couteau (615, 715) utilisé pour guider le couteau le long d'un chemin de coupe prédéfini pour couper le tissu saisi entre les éléments de mâchoire lorsque les éléments de mâchoire sont dans une position fermée ; et **caractérisé par** :
au moins un deuxième élément chauffant se trouvant dans le couteau, ledit deuxième élément chauffant étant une résistance fixe, une résistance variable, un sensistor ou une thermistance.

2. Ensemble d'électrodes selon la revendication 1, dans lequel le premier ou le second élément chauffant est sélectionné dans le groupe consistant en fil de Nichrome, ruban de Nichrome, Calrod et céramique PTC.

3. Ensemble d'électrodes selon la revendication 1 ou 2, comprenant en outre un matériau isolant disposé sur chaque élément de mâchoire, le matériau isolant étant sélectionné dans le groupe consistant en verre, matériaux céramiques et polymériques.

4. Ensemble d'électrodes selon la revendication 1, 2 ou 3, comprenant en outre au moins un capteur configuré pour mesure la température de la au moins une surface d'obturation de tissu électriquement conductrice.

5. Ensemble d'électrodes selon la revendication 1, 2, 3 ou 4, comprenant en outre au moins un capteur configuré pour mesurer la température du tissu.

6. Système pour l'obturation de tissu comprenant :
un ensemble d'électrodes selon l'une quelconque des revendications précédentes ; et
une source (551) d'énergie électrique fonctionnellement connectée à chaque élément de mâchoire, la source d'énergie électrique étant configurée pour fournir de l'énergie électrique à ceux-ci.

7. Système selon la revendication 6, comprenant en outre un mécanisme de contrôle à rétroaction apte à prédire un taux de chauffage.

8. Système selon la revendication 6 ou 7, comprenant en outre au moins un capteur configuré pour mesurer la température de la au moins une surface d'obturation de tissu électriquement conductrice.
